# EUROPÄISCHE PATENTSCHRIFT

(11) **EP 0 516 977 B1**
(45) Veröffentlichungstag und Bekanntmachung des Hinweises auf die Patenterteilung: **26.06.1996**
(21) Anmeldenummer: 92107353.2
(22) Anmeldetag: 30.04.1992
(51) Int. Cl.: G01N 33/18, G01N 21/35

(54) **Anordnung zur Detektion von Fremdstoffen auf Wasseroberflächen**
Arrangement for detecting impurities on water surfaces
Dispositif de détection d'impuretés sur des surfaces d'eau

(30) Priorität: 27.05.1991 DE 4117244
(43) Veröffentlichungstag der Anmeldung: 09.12.1992
(73) Patentinhaber: GRUNDIG E.M.V. Elektro-Mechanische Versuchsanstalt Max Grundig GmbH & Co. KG, D-90762 Fürth (DE)
(72) Erfinder: Hallas, Ernst, Dr. rer. nat., Grundig E.M.V., Kurgartenstrasse 37, W-8510 Fürth/Bay. (DE)

(56) Entgegenhaltungen:
- DE-A- 2 354 125
- DE-A- 2 847 689
- DE-C- 3 438 105
- US-A- 3 364 351
- US-A- 3 803 384

## Beschreibung

Die Erfindung betrifft eine Anordnung zur Detektion von Fremdstoffen auf Wasseroberflächen mit Hilfe einer Lichtabsorptionsmessung.

Insbesondere Flüsse in der Nähe von Industrie- oder Kraftwerksanlagen, in die Abwässer dieser Anlagen eingeleitet werden, müssen regelmäßig oder ständig auf Fremdstoffe untersucht werden. Besonders die Öldetektion spielt hierbei eine große Rolle, da eine Verschmutzung mit Öl weitreichende negative Folgen hat.

Zur Erkennung von Verschmutzungen des Wassers durch Öl oder sonstige Fremdstoffe werden häufig Trübungsmeßgeräte eingesetzt. Dazu werden dem Gewässer Proben entnommen, von denen die Trübungswerte gemessen werden, die auf den Grad der Verschmutzung schließen lassen- Die Art der Verschmutzung kann jedoch mit dieser Methode nicht ermittelt werden, da beispielsweise durch Öl verursachte Verschmutzung die gleichen Trübungswerte liefern kann wie eine durch aufgewühlten Schlamm verursachte Verschmutzung.

Ein Anzeigegerät zum Nachweis der Verschmutzung durch Öl oder ähnliche Verunreinigungen ist in der deutschen Offenlegungsschrift DE 2224310 A1 beschrieben. Dort werden ein oder mehrere Grenzflächen mit einer zu überwachenden Flüssigkeit auf der einen Seite benetzt, während auf der anderen Seite, in einem anderen Medium, ein Strahlenbündel einfällt, dessen Einfallswinkel so gewählt wird, daß er nahe am Grenzwinkel zur Totalreflexion liegt. Bei Vorhandensein von Öl in der zu untersuchenden Flüssigkeit wird der Grenzwinkel überschritten und es findet Totalreflexion statt, so daß eine Signalvorrichtung vom reflektierenden Strahlenbündel betätigt wird.

Bei einem derartigen Anzeigegerät besteht aber der Nachteil, daß jeweils nur eine Probe entnommen wird, die nicht zwingend eine allgemeingültige Aussage über den momentanen Zustand des Gewässers zuläßt.

In der deutschen Offenlegungsschrift DE 3038107 A1 ist ein Verfahren zur berührungsfreien Ermittlung von Öl auf einer Wasseroberfläche beschrieben. Die zu untersuchende Stelle der Wasseroberfläche wird mit einer ultravioletten Lichtquelle bestrahlt, die die Fluoreszenz der erwarteten Ölgruppe anregen soll. Das durch die Fluoreszenzwirkung abgestrahlte Licht wird fotoelektrisch empfangen, wobei die von der Oberfläche reflektierte, durch das UV-Licht der ultravioletten Lichtquelle im wesentlichen sperrende Filter geführte Strahlung, sowie die von der Fluoreszenz herrührende Oberflächenstrahlung vom photoelektrischen Empfänger aufgenommen werden und die Gesamtintensität der ungerichteten Fluoreszenzstrahlung erfaßt wird. Der Nachteil dieses Verfahrens liegt in der punktuellen Messung, bei der nicht eine großflächige Oberfläche überprüft wird, sondern nur ein relativ kleiner Bereich.

Aus der US- A 3 364 351 ist ein Verfahren zur Detektion und Messung von Wasserdampf und Sauerstoff in der Atmosphäre bekannt, bei dem ein Infrarotlichtstrahl über eine Teststrecke von einem Sender zu einem Empfänger geschickt wird, wobei die relativen Intensitäten verschiedener Wellenlängenbänder, die von der Absorption durch Bestandteile der Atmosphäre abhängig sind, bestimmt werden.

Aufgabe der Erfindung ist es, eine Anordnung zur Detektion von Fremdstoffen auf Wasseroberflächen anzugeben, bei dem eine ständige Überwachung eines fließenden Gewässers möglich ist und die Überwachung nicht auf einzelne Proben oder Stellen beschränkt ist.

Diese Aufgabe wird gemäß der Erfindung gelöst, indem die Wasserdampfbildung auf einer Wasseroberfläche ausgewertet wird. Durch Fremdstoffe auf der Wasseroberfläche wird die Dampfbildung der Wasseroberfläche verändert.

Beispielsweise wirkt eine Ölschicht als Feuchtesperre, d.h. an Stellen der Wasseroberfläche, auf denen eine Ölschicht schwimmt, bildet sich kein oder zumindest wesentlich weniger Wasserdampf. Die Bildung von Wasserdampf auf der Oberfläche führt zu Absorptionsverlusten eines Lichtstrahles, insbesondere eines Infrarotlichtrahles, dessen Strahlungsachse etwa parallel zur Wasseroberfläche verläuft. Durch die Messung der Absorptionsverluste kann eine auf der Wasseroberfläche schwimmende Ölschicht erkannt werden.

Die erfindungsgemäße Anordnung zur Messung der Absorption besteht aus einem Lichtsender, vorzugsweise einer Infrarotstrahlungsquelle, die an einem Ufer des fließenden Gewässers angebracht ist. Dieser Lichtsender strahlt in etwa parallel zu Wasseroberfläche in Richtung eines entsprechenden Lichtsensors auf der gegenüberliegenden Seite des Gewässers. Die Anordnung enthält weiterhin eine Einheit zur Messung der Fließgeschwindigkeit des Gewässers und eine Auswerteeinrichtung, mit der die am Sensor aufgenommene Strahlung ausgewertet wird. Die Auswertung erfolgt durch Vergleich mit einem Referenzwert, der aus der Messung an einer Referenzstelle gewonnen wird. Zur Auswertung wird der Wert der Absorption in Beziehung zur Fließgeschwindigkeit des Gewässers gesetzt, so daß auf die Größe der verschmutzten Oberfläche geschlossen werden kann. Der Sender wird von einer Ansteuereinheit angesteuert, die eine konstante Intensität der abgegebenen Lichtstrahlung gewährleistet. Die Absorptionsmessung mittels der erfindungsgemäßen Anordnung wird vorzugsweise im Auslaßkanal für das Abwasser in den Fluß oder ein anderes Gewässer durchgeführt, da in diesem Fall die Distanz zwischen dem Sender und dem Empfänger geringer ist und dort auch nicht mit Störungen durch beispielsweise Schiffahrt zu rechnen ist. Auch natürliche Verschmutzungen der Wasseroberfläche durch Laub oder ähnliches können dort einfach vermieden oder beseitigt werden.

Der Vorteil dieses Verfahrens bzw. der Anordnung zur Durchführung dieses Verfahrens besteht in der Auswertung der gesamten Wasseroberfläche. Es entsteht eine integrierende Wirkung über den gesamten Oberflächenquerschnitt des fließenden Gewässers.

Eine vorteilhafte Ausgestaltung der erfindungsgemäßen Anordnung sieht vor, daß der Lichtsender und der Lichtempfänger auf einer Seite des Gewässers angebracht ist und auf der gegenüberliegenden Seite ein Reflektor angebracht ist. Durch eine derartige Anordnung wird erreicht, daß die aktive Anordnung, die aus der Ansteuerung Auswerteeinheit besteht, nicht räumlich getrennt, sondern auf einer Seite des Gewässers angeordnet ist. Auf der zweiten Seite befindet sich lediglich der Reflektor, der nicht elektrisch angesteuert werden muß.

In einer weiteren Ausgestaltung der Erfindung wird an einer zweiten Stelle, die von einer möglichen Oberflächenverschmutzung des Gewässers durch geeignete Maßnahmen, beispielsweise Filter, geschützt wird, so daß dort immer eine von Fremdstoffen freie Oberfläche gegeben ist, eine Referenzmessung vorgenommen. Der auf diese Weise gewonnene Referenzwert wird ständig der Auswerteeinheit zugeführt. Der Vergleich des Absorptionswertes der zu untersuchenden Wasseroberfläche mit diesem Referenzwert führt zu einem Ergebnis, das unabhängig von äußeren Faktoren, wie zum Beispiel Luftfeuchtigkeit oder Temperatur, ist.

Ein weiterer Vorteil ergibt sich bei der Auswahl der Frequenz des Senders, wenn diese so gewählt wird, daß bei Wasserdampf die Absorption maximal ist. In diesem Fall lassen sich genauere Meßergebnise erzielen, da die Änderung der Absorption bei Vorliegen von Fremdstoffen auf der Wasseroberfläche größer wird.

Eine zusätzliche vorteilhafte Ausgestaltung der Erfindung ergibt sich, wenn mit mehreren definierten Frequenzen eingestrahlt wird, so daß auch eine eingeschränkte Identifikation der Verschmutzungsursache, d.h. des Fremdstoffes auf der Wasseroberfläche, möglich wird. Dies gilt insbesondere für Fremdstoffe, die Gase bilden. Die Einstrahlung mit verschiedenen, definierten Frequenzen kann durch die Wahl der Strahlungsquelle erfolgen, die diese Frequenzen abstrahlt. Es können auch verschiedene

Strahlungsquellen mit jeweils einer definierten Frequenz verwendet werden oder eine breitbandige Strahlungsquelle, der Filter vorgesetzt werden, die nur für bestimmte Frequenzen durchlässig sind.
Durch die Einstrahlung mit verschiedenen Frequenzen wird die Identifikationssicherheit erhöht, wenn beispielsweise eine Frequenz so gewählt wird, daß die Lichtintensität dieser Frequenz durch Wasserdampf maximal verändert wird, wärend die Lichtintensität des Lichts mit der anderen Frequenz durch die Gasphase des Öls maximal verändert wird.

Zum Schutz vor äußeren Einflüssen, wie zum Beispiel Regen, Wind und ähnliches kann die Messanordnung mit einer Abdeckung versehen werden.
Eine Anpassung der Sender-/Empfängereinheit an die Füllstandshöhe des Gewässers ist durch ein geeignetes Nachführsystem möglich- Ein derartiges Nachführsystem erlaubt einen immer gleichen Abstand des Messlichtstrahls von der Wasseroberfläche und somit immer gleiche Messvoraussetzungen.

Eine andere vorteilhafte Ausgestaltung der Erfindung zur Anpassung der Meßanordnung an die Füllstandshöhe des Gewässers ergibt sich, wenn die gesamte Meßanordnung auf einer schwimmfähigen Einrichtung angebracht ist. Durch eine derartige Anordnung ist immer ein gleicher Abstand der Meßanordnung zur Wasseroberfläche gewährleistet.

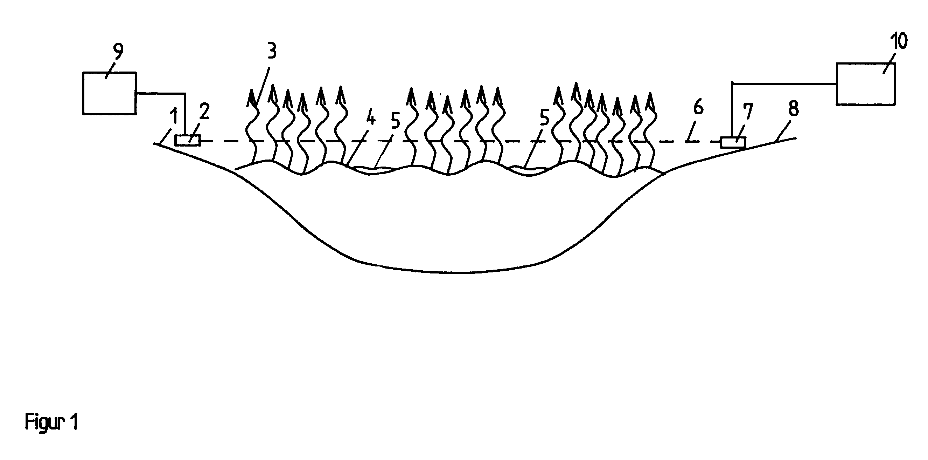

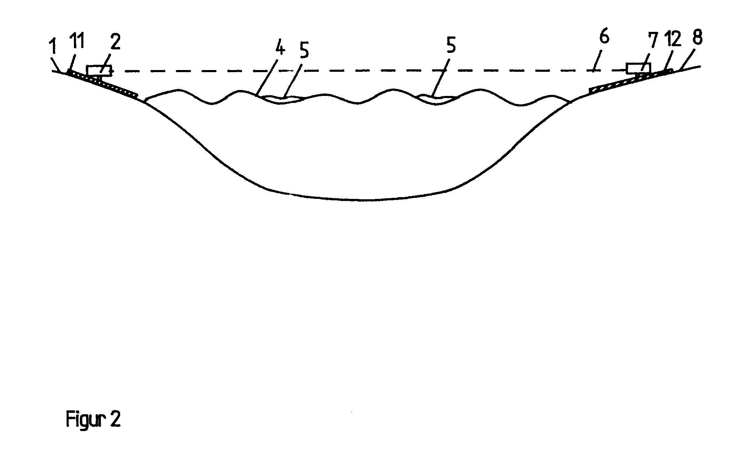

Im folgenden wird die Erfindung an Hand der Figuren 1 und 2 erläutert. Es zeigen:
- Figur 1: eine Anordnung gemäß der Erfindung,
- Figur 2: eine Anordnung gemäß Figur 1 mit einer füllstandsabhängigen Führung.

An einem Ufer 1 des zu untersuchenden Gewässers ist ein Lichtsender 2 angebracht, der vorzugsweise Licht im Infrarotbereich aussendet, wobei die Wellenlänge definiert eingestellt werden kann. Es ist auch die Ausstrahlung von Licht mehrerer Wellenlängen möglich. Bei einem breitbandigen Sender erfolgt die Auswahl bestimmter Wellenlängen durch optische Filter, die im Strahlengang des Senders angeordnet werden. Der Lichtsender 2 wird durch eine Ansteuereinheit 9 angesteuert, die unter anderem die Lichtintensität regelt, so daß die Lichteinstrahlung bei einem Meßzyklus immer mit gleicher Intensität erfolgt. Der Strahlengang 6 des Senders 2 führt etwa parallel zur Wasseroberfläche 4 zum Empfänger 7, der sich am anderen Ufer 8 des Gewässers befindet. Im Strahlengang 6 findet Absorption der Lichtstrahlung durch Wasserdampf statt. In der Figur 1 ist dieser Wasserdampf durch die gewellten Pfeile 3 dargestellt. Durch Fremdstoffe 5 auf der Wasseroberfläche 4 wird die Wasserdampfbildung ganz oder teilweise unterdrückt, so daß durch diese Fremdstoffe, insbesondere durch Öl, die Absorption des Lichtstrahls vermindert wird und diese Verminderung durch die Auswerteeinheit 10, die mit dem Empfänger 7 verbunden ist, festgestellt wird. In der Auswerteeinheit 10 wird die gemessene Lichtintensität mit einem Referenzwert verglichen, der durch eine Referenzmessung gewonnen wird. Vorteilhafterweise wird zur Gewinnung des Referenzwertes in der Nähe des Meßstelle eine weitere Messanordnug aufgebaut, wobei dort die Wasseroberfläche von Fremdstoffen freigehalten wird. Auf diese Weise ist ständig ein Referenzwert verfügbar, der die aktuellen Bedingungen für Wasserdampfbildung, wie z.B. Temperatur, Luftfeuchtigkeit usw., beinhaltet.

Figur 2 zeigt eine Anordnung gemäß Figur 1 mit einem füllstandsabhängigen Nachführungssystem.
Der Sender 2 ist hier auf einer Schiene 11 angebracht, auf der er verschiebbar angeordnet ist. Ebenso ist der Empfänger 7 auf einer entspechenden Schiene 12 angebracht. Bei dieser Anordnung sind der Sender und der Empfänger entsprechend der Füllstandshöhe des Gewässers so verschiebbar, daß der Meßstrahl 6 zur Wasseroberfläche 4 immer den gleichen Abstand hat. Sender und Empfänger werden dabei manuell oder automatisch durch einen motorischen Antrieb verschoben. Bei der automatischen Verschiebung wird ein Füllstandsmeßgerät zur Ermittlung des aktuellen Füllstands verwendet und die Lage von Sender und Empfänger in Abhängigkeit des Meßergebnisses eingestellt.

## Patentansprüche

1. Anordnung zur Detektion von Fremdstoffen auf Wasseroberflächen von offenen, fließenden Wasserläufen mittels Lichtabsorptionsmessung, wobei an den gegenüberliegenden Rändern (1,8) des Wasserlaufes ein Lichtsender (2) und ein Lichtempfänger (7) angebracht sind, deren Strahlungsachse (6) etwa parallel zur Wasseroberfläche (4) verläuft,
**dadurch gekennzeinet, daß**
- der Lichtsender (2) so ausgeführt ist, daß er Strahlung definierter Intensität abgibt,
- der Lichtempfänger (7) so ausgeführt ist, daß er die durch Absorption abgeschwächte Strahlung detektiert,
- eine Einheit zur Messung der Fließgeschwindigkeit des Gewässers vorgesehen ist, und
- die Anordnung eine Auswerteeinheit (10) enthält, die an den Empfänger angeschlossenen ist, und in der die Meßergebnisse in Abhängigkeit von der Fließgeschwindigkeit des Gewässers ausgewertet werden.

2. Anordnung nach Anspruch 1,
**dadurch gekennzeichnet,** daß der Lichtsender (2) und der Lichtempfänger (7) auf einer Seite des Gewässers angeordnet sind und auf der gegenüberliegenden Seite ein Reflektor angeordnet ist, der den Lichtstrahl des Lichtsenders (2) reflektiert.

3. Anordnung nach Anspruch 1 oder 2,
**dadurch gekennzeichnet**, daß
die Auswerteeinheit (10) so ausgeführt ist, daß das Ergebnis einer Referenzmessung zur Bestimmung des Absorptionsverhaltens an anderer Stelle als Vergleichswert zugeführt werden kann.

4. Anordnung nach einem der Ansprüche 1 bis 3,
**dadurch gekennzeichnet,** daß
der Lichtsender (2) so ausgeführt ist, daß er bei einer ausgewählten Frequenz einstrahlt, die durch Wasserdampf besonders gut absorbiert wird.

5. Anordnung nach einem der Ansprüche 1 bis 4,
**dadurch gekennzeichnet,** daß
der Lichtsender (2) mit mehreren Frequenzen einstrahlt, um die Ursache der Absorptionsänderung in Abhängigkeit der eingestrahlten Frequenz zu bestimmen.

6. Anordnung nach Anspruch 4 oder 5,
**dadurch gekennzeichnet,** daß
der Lichtsender (2) breitbandig strahlt und ihm optische Filter vorgelagert sind, die nur für eine oder mehrere ausgewählte Frequenzen durchlässig sind.

7. Anordnung nach einem oder mehreren der Ansprüche 1 bis 6, **dadurch gekennzeichnet,** daß zum Schutz vor äußeren Einflüssen die gesamte Anordnung mit einer Abdeckung versehen ist.

8. Anordnung nach einem oder mehreren der Ansprüche 1 bis 7, **dadurch gekennzeichnet,** daß der Lichtsender (2) und der Lichtempfänger (7) auf Führungsschienen (11,12) angeordnet sind und entlang der Schienen verschoben werden können, so daß der Abstand des Meßstrahles (6) von der Wasseroberfläche (4) konstant ist.

9. Anordnung nach einem oder mehreren der Ansprüche 1 bis 8, **dadurch gekennzeichnet,** daß die Meßanordnung auf einer schwimmfähigen Vorrichtung angeordnet ist.

## Claims

1. Arrangement for the detection of extraneous substances on water surfaces of open flowing water courses by means of light absorption measurement, in which a light transmitter (2) and a light receiver (7) whose radiation axis (6) extends approximately parallel to the water surface (4) are mounted at oppositely situated edges (1, 8) of the water course, characterized in that
- the light transmitter (2) is constructed so that it delivers radiation of a defined intensity,
- the light receiver (7) is constructed so that it detects the radiation attenuated by absorption,
- a unit is provided for the measurement of the flow rate of the body of water, and
- the arrangement contains an evaluation unit (10) which is connected to the receiver and in which the measurement results are evaluated as a function of the flow rate of the body of water.

2. Arrangement according to Claim 1, characterized in that the light transmitter (2) and the light receiver (7) are disposed on one side of the body of water and a reflector which reflects the light beam of the light transmitter (2) is disposed on the oppositely situated side.

3. Arrangement according to Claim 1 or 2, characterized in that the evaluation unit (10) is constructed so that the result of a reference measurement can be supplied as a comparison value to determine the absorption behaviour at another point.

4. Arrangement according to one of Claims 1 to 3, characterized in that the light transmitter (2) is constructed so that it radiates at a selected frequency which is absorbed particularly well by water vapour.

5. Arrangement according to one of Claims 1 to 4, characterized in that the light transmitter (2) radiates at a plurality of frequencies in order to determine the cause of the change in absorption as a function of the radiated frequency.

6. Arrangement according to Claim 4 or 5, characterized in that the light transmitter (2) radiates in a broad-band fashion and optical filters are placed in front of it which are transparent only for one or more selected frequencies.

7. Arrangement according to one or more of Claims 1 to 6, characterized in that the entire arrangement is provided with a cover as a protection against external influences.

8. Arrangement according to one or more of Claims 1 to 7, characterized in that the light transmitter (2) and the light receiver (7) are disposed on guide rails (11, 12) and can be displaced along the rails so that the distance of the measurement beam (6) from the water surface (4) is constant.

9. Arrangement according to one or more of Claims 1 to 8, characterized in that the measurement arrangement is disposed on a buoyant device.

## Revendications

1. Agencement de détection d'impuretés à la surface de cours d'eaux vives ouvertes, par mesure de l'absorption de lumière, un émetteur de lumière (2) et un récepteur de lumière (7) étant installés sur les rives opposées (1, 8) du cours d'eau, leur axe de rayonnement (6) s'étendant à peu près parallèlement à la surface (4) de l'eau,
caractérisé en ce que:
- l'émetteur de lumière (2) est réalisé de telle sorte qu'il émette un rayonnement d'une intensité définie,
- le récepteur de lumière (7) est réalisé de telle sorte qu'il détecte le rayonnement affaibli par l'absorption,
- une unité de mesure de la vitesse d'écoulement de l'eau est prévue, et que
- l'agencement comporte une unité d'analyse (10) raccordée au récepteur, dans laquelle on analyse les résultats de mesure en fonction de la vitesse d'écoulement de l'eau.

2. Agencement selon la revendication 1, caractérisé en ce que l'émetteur de lumière (2) et le récepteur de lumière (7) sont disposés d'un côté de l'eau, et un réflecteur réfléchissant le rayon lumineux de l'émetteur de lumière (2) est disposé du côté opposé.

3. Agencement selon la revendication 1 ou 2, caractérisé en ce que l'unité d'analyse (10) est conçue de manière telle que ledit résultat puisse être soumis à une comparaison avec une mesure de référence destinée à déterminer le comportement d'absorption à un autre endroit.

4. Agencement selon l'une des revendications 1 à 3, caractérisé en ce que l'émetteur de lumière (2) est conçu de manière telle qu'il émette une fréquence sélectionnée, qui subit une absorption particulièrement forte par la vapeur d'eau.

5. Agencement selon l'une des revendications 1 à 4, caractérisé en ce que l'émetteur de lumière (2) émet plusieurs fréquences, de manière à pouvoir déterminer la cause du changement d'absorption en fonction de la fréquence incidente.

6. Agencement selon la revendication 4 ou 5, caractérisé en ce que l'émetteur de lumière (2) émet en large bande, et que des filtres optiques sont placés devant l'émetteur, qui ne sont passants que pour une ou plusieurs des fréquences sélectionnées.

7. Agencement selon l'une ou plusieurs des revendications 1 à 6, caractérisé en ce que tout le dispositif est pourvu d'une couverture destinée à le protéger contre les influences extérieures.

8. Agencement selon l'une ou plusieurs des revendications i à 7, caractérisé en ce que l'émetteur de lumière (2) et le récepteur de lumière (7) sont disposés sur des rails de guidage (11, 12) et peuvent être déplacés le long de ces rails, de manière que la distance entre le rayon de mesure (6) et la surface de l'eau (4) reste constante.

9. Agencement selon l'une ou plusieurs des revendications 1 à 8, caractérisé en ce que l'agencement de mesure est disposé sur un engin susceptible de flotter.
